(19) Europäisches Patentamt
European Patent Office
Office européen des brevets

(11) Numéro de publication: 0 356 270 A2

(12) **DEMANDE DE BREVET EUROPEEN**

(21) Numéro de dépôt: 89401952.0

(22) Date de dépôt: 06.07.89

(51) Int. Cl.[5]: C 07 C 57/12
C 07 D 295/08,
C 07 D 213/67,
C 07 D 311/72,
C 07 C 219/08, A 61 K 31/23,
A 61 K 31/44, A 61 K 31/355,
A 61 K 31/50

(30) Priorité: 06.07.88 FR 8809172

(43) Date de publication de la demande:
28.02.90 Bulletin 90/09

(84) Etats contractants désignés:
AT BE CH DE ES FR GB GR IT LI LU NL SE

(71) Demandeur: Azar, Roger F.
6 Avenue F. Le Play
F-75007 Paris (FR)

Grinda, Jean Robert
14 Rue Cassette
F-75006 Paris (FR)

(72) Inventeur: Azar, Roger F.
6 Avenue F. Le Play
F-75007 Paris (FR)

Grinda, Jean Robert
14 Rue Cassette
F-75006 Paris (FR)

(74) Mandataire: Burtin, Jean-François
Bugnion Associés Département GEF1B 55 Rue
Boissonade
F-75014 Paris (FR)

Revendications pour les Etats contractants suivants: ES + GR.

(54) **Nouveaux esters d'acide gras leurs procédés d'obtention et leurs emplois industriels.**

(57) La présente invention se rapporte à de nouveaux esters de l'acide gamma linolénique et à leurs procédés d'obtention.

Elle a spécifiquement pour objet des esters aromatiques ou aliphatiques de l'acide gamma linolénique répondant à la formule générale I

R - O$(CH_2)n_1$-$(CH_2)n_2$- Z (I)

dans laquelle

R est le reste acyle de l'acide γ-linolénique ou (cis 6, 9,12-octadecatrienoique)

$n_1$ est un nombre entier variant de 0 à 5

$n_2$ est un nombre entier variant de 0 à 2

Z est de l'hydrogène, un reste aminé de formule génerale

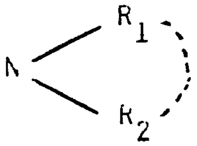

dans laquelle

$R_1$ et $R_2$ identiques ou différents, représentent de l'hydrogène, un radical alcoyle inférieur, alcenyle inférieur, aralcoyle inferieur, cycloalcoyle inférieur ou bien, forment avec l'atome d'azote adjacent un hetérocycle azoté ayant de 4 à 7 maillons pouvant inclure un autre hétéroatome choisi dans le groupe constitué par de l'oxygène, du soufre ou un radical imino

> N - $R_3$

dans lequel

$R_3$ est un radical alcoyle inférieur non substitué ou substitué par un hydroxyle libre ou bloqué par alcoylation ou estérification le reste pyridoxyle

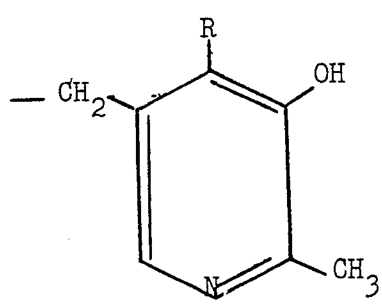

EP 0 356 270 A2

dans lequel
R est un radical formyle, hydroxy méthyle, amino méthyle ou un carboxyle
et le reste tocophéryle

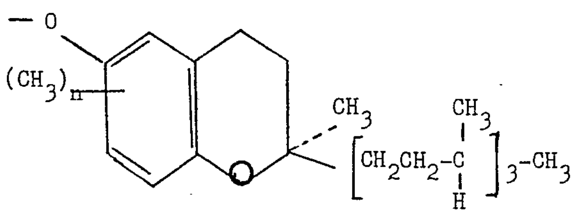

dans laquelle
n est un nombre entier variant de 1 à 3
avec la restriction que lorsque $n_1$ est égal à un et $n_2$ est égal à zéro Z est différent de l'hydrogène.
ainsi que les sels d'amino esters avec un acide minéral ou organique.

L'invention concerne également des procédés d'obtention des composés de formule générale I.

Les nouveaux esters trouvent un emploi en biologie, en cosmétologie ou en thérapeutique.

## Description

## NOUVEAUX ESTERS D'ACIDE GRAS LEURS PROCEDES D'OBTENTION ET LEURS EMPLOIS INDUSTRIELS

La présente invention se rapporte à de nouveaux esters d'acide gras et, plus particulièrement, à des esters d'acide γ-linolénique.

Elle a plus précisément pour objet des esters d'alcoyle substitués ou non substitués de l'acide γ-linolénique.

Elle a spécifiquement pour objet des esters aromatiques ou aliphatiques, éventuellement substitués par un reste azoté, de l'acide γ-linolénique répondant à la formule générale I

$R - O\ (CH_2)_{n1} - (CH_2)_{n2}\ Z$ (I)

dans laquelle

R est le reste acyle de l'acide γ-linolénique ou (cis 6,9,12-octadecatrienoïque)

$n_1$ est un nombre entier variant de 0 à 5

$n_2$ est un nombre entier variant de 0 à 2

Z est de l'hydrogène, un reste aminé de formule générale

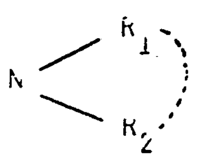

dans laquelle

$R_1$ et $R_2$ identiques ou différents, représentent de l'hydrogène, un radical alcoyle inférieur, alcenyle inférieur, aralcoyle inférieur, cycloalcoyle inférieur, ou bien, forment avec l'atome d'azote adjacent un hetérocycle azoté ayant de 4 à 7 maillons pouvant inclure un autre hétéroatome choisi dans le groupe constitué par de l'oxygène, du soufre ou un radical imino

$> N - R_3$

dans lequel

$R_3$ est un radical alcoyle, inférieur non substitué ou substitué par un hydroxyle libre ou bloqué par alcoylation ou estérification,

le reste pyridoxyle

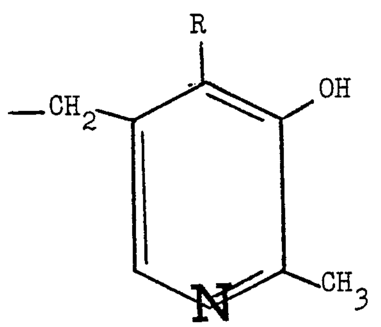

dans lequel

R est un radical formyle, hydroxy méthyle, amino méthyle ou un carboxyle

et le reste tocophéryle

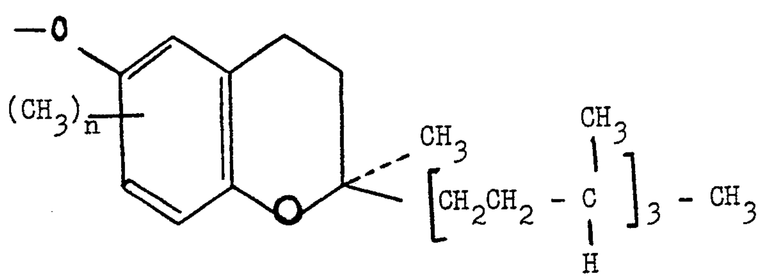

dans laquelle

n est un nombre entier variant de 1 à 3

avec la restriction que lorsque $n_1$ est égal à un et $n_2$ est égal à zéro, Z est différent de l'hydrogène.

Parmi les composés de formule générale 1, on distinguera quatre sous-groupes actuellement préférés :

- les esters d'alcoyle de l'acide γ-linolénique tels que l'ester de butyle, de pentyle, de terbutyle ou d'éthyle

($n_1$ = 1 - 5 Z = H $n_2$ = 0)
- les esters d'amino alcools et notamment les esters de diméthyl amino éthyle, de diethylamino éthyle, de dipropylamino propyle, de dibutylamino éthyle, de morpholino ethyle, de N-méthyl pipérazinyl éthyle, de N-(méthoxyethyl piperazinyl) ethyl et les esters semblables. ($n_1$ = zéro $n_2$ = 2)

L'invention comprend aussi les sels des esters d'amino alcools avec un acide minéral ou organique physiologiquement compatible. Ces sels constituent des formes d'utilisation préférés car ce sont des solides cristallisables ce qui permet ou facilite leur purification.
- les esters de pyridoxyle et notamment l'ester de pyridoxal, l'ester de pyridoxol ou l'ester de pyridoxamine. ($n_1$ = 0 $n_2$ = zero)
- les esters de tocophéryle et notamment l'ester d'α-tocophérol, l'ester de β-tocophérol et l'ester de γ-tocophérol. ($n_1$ et $n_2$ = zero)

Les composés de formule générale 1 selon l'invention peuvent être obtenus par différents procédés :
- par transestérification en milieu acide entre le γ-linolénate de méthyle et un acétate du composé hydroxylé de formule
$CH_3$ COO $(CH_2)_{n1}$ - $(CH_2)_{n2}$ - Z
dans laquelle $n_1$, $n_2$ et Z ont les définitions antérieures.
- par action de l'acide γ-linolénique sur un oxyde d'alcoylène en milieu acide pour former un ester d'hydroxy alcoyle, que l'on chlore pour former l'ester de chloroalcoyle puis le condense avec une amine primaire ou secondaire de formule

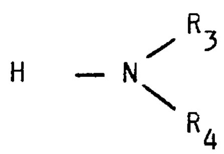

pour obtenir un ester d'amino alcool
- par action d'un phénol ou d'un alcanol sur le γ-linolénate de méthyle, comme le pyridoxol ou le tocophérol, en milieu acide ou en milieu alcalin.

Selon la nature de l'alcool ou du phénol, l'une de ces voies de synthèse sera choisie car le γ-linolénate de méthyle qui sert de matière première s'altère très rapidement par chauffage ou oxydation et il importe que la réaction soit conduite dans des conditions où de telles transformations seront réduites au minimum car la purification des produits finaux est souvent difficile à réaliser.

L'agent acide qui catalyse la transestérification est de préférence un acide minéral comme l'acide sulfurique ou l'acide per chlorique.

L'action du γ-linolénate de méthyle ou de l'acide γ-linolénique sur un oxyde d'alcoylène comme l'oxyde d'éthylène, l'oxyde de propylène ou l'oxyde de butylène conduit aux esters d'hydroxy alcoyle correspondants. La réaction s'effectue en présence d'un acide comme l'acide acétique ou l'acide méthane sulfonique.

Les esters de chloroalcoyle formés par action de l'acide chlorhydrique sont des solides qui cristallisent aisément. Ils permettent l'isolement d'un intermédiaire de synthèse pur. Ils sont convertis en dérivés aminés soit par addition d'un excès d'amine qui sert de solvant, soit par addition de la quantité théorique d'amine préalablement dissoute dans un solvant inerte comme le diméthyl sulfoxyde, l'acétonitrile, le dioxane, le tétrahydrofuran ou le chlorure de méthylène.

Les esters ainsi obtenus constituent des matières premières précieuses soit pour la purification de l'acide γ-linolénique lui-même soit comme substrat biologique pour la conversion par la voie enzymatique en dérivés homo- et dihomolinoléniques, soit encore par eux-même comme produits de cosmétologie. Ils peuvent, selon leur degré de solubilité dans les milieux aqueux, trouver un emploi en thérapeutique comme précurseurs naturels des prostaglandines et des prostacyclines.

Les exemples suivants illustrent l'invention sans toutefois la limiter.

## EXEMPLE I

### γ-Linolénate de morpholinoéthyle

STADE A :

dans un becher de 100 ml on introduit 6.55 g (0.05 mol) de β-morpholinoéthanol dans 20 ml environ de toluène. On place sous agitation et on ajoute peu à peu 4.2 g (soit 3.8 ml) de chlorure d'acétyle (0.05 mol + 10 % d'excès) préalablement dilué dans 20 ml de toluène. Le mélange se prend en masse. On constate une légère montée en température. Ensuite, on laisse refroidir à température ordinaire et on sépare le précipité puis on essore sur verre fritté, on lave avec 10 ml de toluène puis on sèche le précipité de chlorhydrate d'acétyl morpholinoéthanol au dessicateur.

STADE B :

Dans un ballon tricol de 100 ml, on prépare du méthylate de sodium au départ de 0.50 g de sodium et de méthanol. On évapore l'excès de méthanol. On ajoute 5.85 g de γ-linolénate de méthyle préalablement dissout

dans 15ml d'acétone anhydre, puis 4.19 g du chlorhydrate de N-acétyl morpholinoéthanol. On porte le mélange au reflux pendant 1 h 30 sous azote. On filtre ensuite, puis on essore pour séparer les sels minéraux. Le solvant est évaporé sous pression réduite et on récupère le γ-linolénate de morpholino éthyle qui s'est formé.

**EXEMPLE II**

**γ-Linolénate de Butyle**

Dans un ballon de 100 ml, on introduit 10.8 g de n-butanol, 1 goutte d'acide sulfurique et 4 g de linolénate de méthyle pur. On porte au reflux pendant 1 h 30. On laisse refroidir puis on purifie le produit réactionnel par distillation fractionnée sous vide. On élimine ainsi le butanol en excès puis le γ-linolénate de méthyle qui n'a par réagi. Le résidu est repris dans l'acétonitrile, neutralisé avec une solution saturée de bicarbonate de sodium puis extrait à trois reprises au chlorure de méthylène. Les phases chlorométhyléniques sont réunies, lavées à l'eau puis séchées sur sulfate de sodium, filtrées et évaporées à sec sous vide.

Le γ-linolénate de butyle est un liquide huileux, monotache en chromatographie en couche mince.

**EXEMPLE III**

**γ-Linolénate de pyridoxol**

Au départ de 5.85 g de γ-linolénate de méthyle et de 3.70 g d'acétate de pyridoxole dans l'acétone, ou ajoute quelques mg de méthylate de sodium sec préparé par action du sodium sur le méthanol. On dilue le mélange avec 25 ml d'acétone et porte le tout au reflux du solvant pendant 4 heures. Après refroidissement, on filtre, évapore le solvant sous vide et récupère le γ-linolénate de pyridoxol.

**EXEMPLE IV**

**γ-Linolénate de (N-méthyl pipérazinyl-1 éthyle)**

Stade A :γ-linolénate de β-chloroéthyle

On introduit dans un erlenmeyer de 100 ml, 12 g de γ-linolénate de méthyle, 25 ml de chlorhydrine du glycol et 1 ml d'acide perchlorique. On porte le mélange au reflux pendant 3 heures (130°C environ), qui prend une forte coloration. On laisse ensuite refroidir et on chasse par distillation l'excès de chlorhydrine. On lave le résidu à trois reprises avec 20 ml d'eau après l'avoir dilué avec 50 ml d'éther.

On lave encore avec deux portions de 20 ml d'eau jusqu'à pH 6. On sèche alors l'éther sur sulfate de sodium, décolore au noir, filtre et évapore à sec l'éther. On obtient un résidu solide pesant environ 10 g

Par chromatographie sur plaque, on détecte la présence de 30 % environ de γ-linolénate de méthyle qui n'a pas réagi (Rf. 0,42) et de 60 % de l'ester de β-chloroéthyle (Rf. 0,55).

STADE B :

5 g du mélange obtenu au stade A correspondant à 0,008 mol d'ester de β-chloroéthyle sont additionnés de 1 g de N-méthyl piperazine et de 20 ml d'isopropanol. On porte le mélange au reflux pendant 2 heures puis on évapore l'isopropanol. Le résidu qui ne cristallise pas est alors repris par 50 ml d'éther anhydre. Il apparait un précipité qu'on laisse reposer une nuite en glacière. On le sépare par filtration, l'essore et le recristallise immédiatement du mélange cyclohexane (1)-éther isopropylique (2) après traitement au noir active.

On obtient ainsi le γ -linolénate de (N-méthyl piperazinyl-1) éthyle sous forme de cristaux gras fondant à 135°C environ.

**EXEMPLE V**

**γ-linolénate d'α-tocophéryle**

Dans un ballon tricol de 250 ml, on prépare du méthylate de sodium par réaction de 0,25 g environ de sodium sur 5 ml de méthanol. Après achèvement de la réaction, on évapore le méthanol en évitant toute entrée d'humidité. On ajoute au méthylate une solution préalablement préparée de 2,92 g de γ-linolénate de méthyle dans 10 ml d'acétone sec, puis une solution de 4,73 g d'acétate d' α-tocophérol dans 10 ml d'acétone sec ajoutée très progressivement. On installe une amenée d'azote puis porte le mélange au reflux du solvant (soit environ 65°C) pendant une heure. On filtre ensuite sur verre fritté et essore le précipité minéral. La solution acétonique est évaporée à sec. Le résidu est repris par 10 ml de chlorure de méthylène et additionné de 15 ml de méthanol. Par repos en glacière, le γ -linolénate d' α-tocophéryle se sépare sous forme de pâte. On le filtre, l'essore à fond et le cristallise du méthanol par chaud et froid.

Le produit est caractérisé par son Rf en chromatographie en couche mince.

**Revendications**

1. De nouveaux esters aromatiques ou aliphatiques de l'acide γ-linolénique répondant à la formule générale I

$R - O\,(CH_2)_{n1} - (CH_2)_{n2}\,Z$ (I)

dans laquelle

R est le reste acyle de l'acide γ-linolénique ou (cis ,cis,cis octadeca 6, 9, 12-trienoïque)

$n_1$ est un nombre entier variant de 0 à 5

$n_2$ est un nombre entier variant de 0 à 2

Z est de l'hydrogène, un reste aminé de formule générale

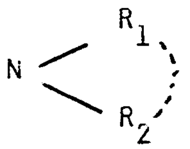

dans laquelle

$R_1$ et $R_2$ identiques ou différents, représentent de l'hydrogène, un radical alcoyle inférieur, alcenyle inférieur, aralcoyle inférieur, cycloalcoyle inférieur ou bien, forment avec l'atome d'azote adjacent un hétérocycle azoté ayant de 4 à 7 maillons pouvant inclure un autre hétéroatome choisi dans le groupe constitué par de l'oxygène, du soufre ou un radical imino

$> N - R_3$

dans lequel

$R_3$ est un radical alcoyle inférieur non substitué ou substitué par un hydroxyle libre ou bloqué par alcoylation ou estérification

le reste pyridoxyle

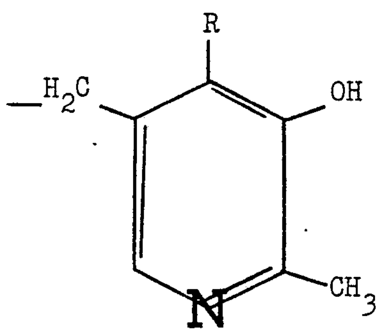

dans lequel

R est un radical formyle, hydroxy méthyle, amino méthyle ou un carboxyle

et le reste tocophéryle

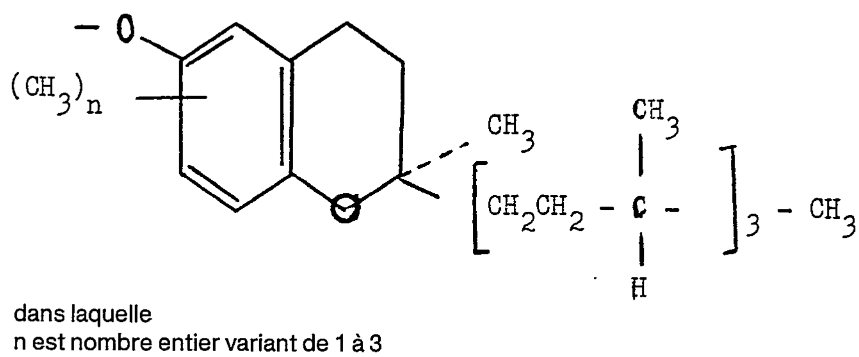

dans laquelle

n est nombre entier variant de 1 à 3

$n_2$ est égal à 0 ou 1

avec la restriction que lorsque $n_1$ est égal à un et que $n_2$ est égal à zéro Z est différent de l'hydrogène.

2. Les esters d'alcoyle de l'acide γ-linolénique selon la revendication 1 pour lesquels Z est de l'hydrogène, $n_1$ est un nombre entier variant de 0 à 5 et $n_2$ est égal à zéro ou un avec la restriction que la somme $n_1$ plus $n_2$ est plus grande que un.

3. Les esters d'amino alcoyle de l'acide γ-linolénique selon la revendication 1 pour lesquels Z est un radical amine de formule

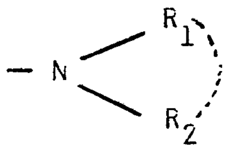

dans laquelle $R_1$ et $R_2$ ont les significations fournies antérieurement et la somme $n_1$ plus $n_2$ est plus grande que zéro

4. Les esters de tocophéryle de l'acide γ-linolénique selon la revendication 1 répondant à la formule générale

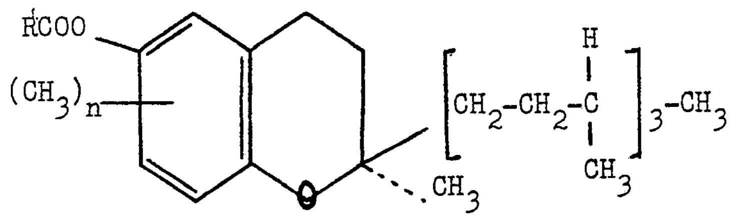

dans laquelle n est égal à 1,2 ou 3.
R est défini comme précédemment

5. Les esters de pyridoxyle de l'acide γ-linolénique selon la revendication 1 répondant à la formule générale

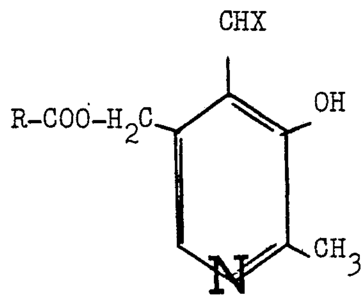

dans laquelle X représente un oxygène, un hydroxyle libre ou estérifié ou un reste aminé.

6. Les sels des esters d'amino alcools selon la revendication 3 avec un acide minéral ou organique physiologiquement compatible.

7. Un composé selon l'une des revendications 1 à 6 à savoir le γ-linolénate d'α-tocophérol.

8. Un composé selon l'une des revendications 1 à 6, à savoir le γ-linolénate de diethylamino éthyle et son chlorhydrate.

9. Un composé selon l'une des revendications 1 à 6, à savoir le γ-linolénate de (N-méthylpiperazinyl) éthyle et son chlorhydrate.

10. Un composé selon l'une des revendications 1 à 6, à savoir le γ-linolénate de morpholino éthyle.

11. Un composé selon l'une des revendications 1 à 6, à savoir le γ-linolénate de pyridoxol.

12. Un procédé pour obtenir les esters aromatiques ou aliphatiques de l'acide γ-linolénique répondant à la formule générale I

$R - O(CH_2)_{n1} - (CH_2)_{n2} - Z$ (I)

dans laquelle R est le reste acyle de l'acide γ-linolénique et les substituants $n_1$, $n_2$ et Z sont définis comme précédemment
qui consiste en ce que l'on soumet le γ-linolénate de méthyle à l'action d'un acétate d'aryle ou d'alcoyle de formule générale

$CH_3\,COO\,(CH_2)_n - (CH_2)_n - Z$ (I)

dans laquelle $n_1$, $n_2$ et Z sont définis comme précédemment en présence d'un acide minéral, pour obtenir l'ester désiré.

**Revendications pour les Etats contractants suivants: ES, GR**

1° - Un procédé pour obtenir les esters aromatiques ou aliphatiques de l'acide γ-linolénique répondant à la formule générale I

$R - O(CH_2)_{n1} - (CH_2)_{n2} - Z$ (I)

dans laquelle
R est le reste acyle de l'acide γ-linolénique ou (cis, cis, cis octadeca 6,9,12-trienoïque)
$n_1$ est un nombre entier variant de 0 à 5

$n_2$ est un nombre entier variant de 0 à 2
Z est de l'hydrogène, un reste aminé de formule générale

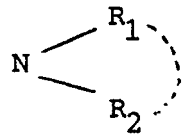

dans laquelle
$R_1$ et $R_2$ identiques ou différents, représentent de l'hydrogène, un radical alcoyle inférieur, alcenyle inférieur, aralcoyle inférieur, cycloalcoyle inférieur ou bien, forment avec l'atome d'azote adjacent un hétérocycle azoté ayant de 4 à 7 maillons pouvant inclure un autre hétéroatome choisi dans le groupe constitué par de l'oxygène, du soufre ou un radical imino
$> N - R_3$
dans lequel
$R_3$ est un radical alcoyle inférieur non substitué ou substitué par un hydroxyle libre ou bloqué par alcoylation ou estérification;
le reste pyridoxyle

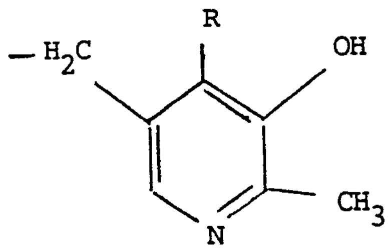

dans lequel
R est un radical formyle, hydroxy méthyle, amino méthyle ou un carboxyle;
et le reste tocophéryle

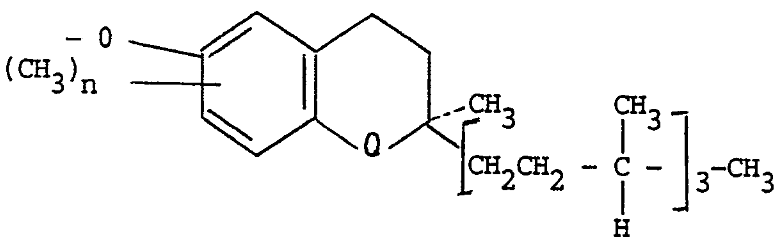

dans lequel
n est un nombre entier variant de 1 à 3
$n_2$ est égal à 0 ou 1
avec la restriction que lorsque $n_1$ est égal à un et que $n_2$ est égal à zéro Z est différent de l'hydrogène qui consiste en ce que l'on soumet le $\gamma$-linolénate de méthyle à l'action d'un acétate d'aryle ou d'alcoyle de formule générale
$CH_3COO(CH_2)_{n1} - (CH_2)_{n2} - Z$
dans laquelle $n_1$, n2 et Z sont définis comme précédemment en présence d'un acide minéral, pour obtenir l'ester désiré.